# EUROPEAN PATENT APPLICATION

(11) **EP 4 238 485 A1**
(43) Date of publication of application: **06.09.2023**
(21) Application number: 21886251.4
(22) Date of filing: 27.10.2021
(51) Int. Cl.: A61B 3/135

(54) **MEDICAL EXAMINATION DEVICE**

(30) Priority: 28.10.2020 JP 2020180487
(71) Applicant: KOWA COMPANY, LTD., Naka-ku Nagoya-shi Aichi 460-8625 (JP)
(72) Inventor: KAMBE Naoki, Kawasaki-shi Kanagawa 216-0004 (JP); KAKUUCHI Atsushi, Chofu-shi Tokyo 182-0021 (JP)
(74) Representative: Pitchford, James Edward
(86) International application number: PCT/JP2021/039585
(87) International publication number: WO 2022/092133

(57) **Abstract**

In order to fix a position of a gripping hand to enable stable operation for examination, a medical examination device having a grip portion that is grippable by an operator in such a manner as to be sandwiched between a thumb and at least one of four fingers including an index finger, a middle finger, a ring finger, and a little finger on an index finger side of one hand, to examine a subject by performing operation in a state where the grip portion is gripped with the one hand, includes a finger hook portion provided on the grip portion to be hooked on any of the fingers located on the index finger side when the grip portion is gripped by the operator with the one hand. In addition, in the medical examination device, an index finger-side operation unit used for examination operation may be provided at a position that is reachable by a finger assumed to be used for the operation in advance as a finger of which a position is fixed by the finger hook portion when the operator grips the grip portion with the one hand among the index finger-side fingers.

## Description

### Technical Field

The present invention relates to a medical examination device for examining a subject.

### Background Art

Conventionally, in the medical field, a handheld examination device for examining a subject such as a human or an animal has been used. Patent Literature 1 discloses an examination device capable of examining an anterior ocular segment by irradiating an eye, which is a subject, with slit light while being held by an operator with one hand.

### Citation List

### Patent Literature

Patent Literature 1: JP 4136620 B2

### Summary of Invention

### Technical Problem

Here, in the examination device described in Patent Literature 1, since a component for fixing a position of a gripping hand is not provide, there has been concern that, for example, when an operator grasps the examination device without a firm visual check, the position of the hand may be deviated from an ideal position for operation, which makes it difficult to operate the examination device.

The present invention has been made in view of the aforementioned problem, and an object of the present invention is to provide a medical examination device capable of fixing a position of a gripping hand to enable stable operation for examination.

### Solution to Problem

A medical examination device having a grip portion that is grippable by an operator in such a manner as to be sandwiched between a thumb and at least one of four fingers including an index finger, a middle finger, a ring finger, and a little finger on an index finger side (hereinafter referred to as "index finger-side fingers") of one hand, to examine a subject by performing operation in a state where the grip portion is gripped with the one hand, according to the present invention includes a finger hook portion provided on the grip portion to be hooked on any of the fingers located on the index finger side when the grip portion is gripped by the operator with the one hand.

In addition, in the medical examination device according to the present invention, an index finger-side operation unit used for examination operation may be provided at a position that is reachable by a finger assumed to be used for the operation in advance as a finger of which a position is fixed by the finger hook portion when the operator grips the grip portion with the one hand among the index finger-side fingers.

In addition, in the medical examination device according to the present invention, assuming that a finger closer to the thumb than a finger receiving a load from the finger hook portion is an operation finger, the index finger-side operation unit may be provided at a position that is reachable by the operation finger.

In addition, in the medical examination device according to the present invention, a thumb-side operation unit used for examination operation may be provided at a position that is reachable by the thumb of which a position is fixed by the finger hook portion when the operator grips the grip portion with the one hand.

In addition, the medical examination device according to the present invention may further include: an irradiation unit that irradiates the subject with irradiation light; an irradiation angle adjustment unit that adjusts an irradiation angle of the irradiation light by rotating the irradiation unit about a rotation shaft; and a scale portion that provides scales each indicating a rotation angle on an upper surface of the rotation shaft.

In addition, in the medical examination device according to the present invention, a load balance may be configured so that a center of the balance of the entire device is located on the index finger side when the grip portion is divided into two sides, a thumb side and the index finger side, by a reference plane that is a virtual plane parallel to a central axis of the grip portion and passes a position of the finger hook portion.

### Advantageous Effects of Invention

One or more deficiencies are solved by the embodiments of the present application.

### Brief Description of Drawings

Fig. 1 is an external view illustrating an example of an external appearance of a configuration of a medical examination device 100 corresponding to at least one of embodiments of the present invention.
Fig. 2 is an external view illustrating the example of the external appearance of the configuration of the medical examination device 100 corresponding to at least one of embodiments of the present invention.
Fig. 3 is an external view illustrating the example of the external appearance of the configuration of the medical examination device 100 corresponding to at least one of embodiments of the present invention.
Fig. 4 is an external view when an operator grips the medical examination device 100 corresponding to at least one of embodiments of the present invention with one hand.
Fig. 5 is an explanatory diagram for explaining operation buttons used for examination operation in a thumb-side operation unit of the medical examination device 100 corresponding to at least one of embodiments of the present invention.
Fig. 6 is an explanatory diagram for explaining operation buttons used for examination operation in a thumb-side operation unit of the medical examination device 100 corresponding to at least one of embodiments of the present invention.
Fig. 7 is an explanatory diagram for explaining operation buttons used for examination operation in a thumb-side operation unit of the medical examination device 100 corresponding to at least one of embodiments of the present invention.
Fig. 8 is an explanatory diagram for explaining operation buttons used for examination operation in a thumb-side operation unit of the medical examination device 100 corresponding to at least one of embodiments of the present invention.
Fig. 9 is an explanatory diagram for explaining an example of a shape of a finger hook portion of the medical examination device 100 corresponding to at least one of embodiments of the present invention.
Fig. 10 is an explanatory diagram for explaining an example of a shape of a finger hook portion of the medical examination device 100 corresponding to at least one of embodiments of the present invention.
Fig. 11 is an explanatory diagram for explaining an example of a scale portion 90 of the medical examination device 100 corresponding to at least one of embodiments of the present invention.

### Description of Embodiments

Hereinafter, an example of a medical examination device according to an embodiment of the present invention will be described with reference to the drawings. For example, the medical examination device is a device called a so-called slit lamp that irradiates an eye (hereinafter, referred to as an "eye to be examined"), which is an example of a subject, with slit light to observe scattered light generated in the eye to be examined, thereby examining a cornea, a crystalline lens, or the like of the eye to be examined. Other examples of the medical examination device include ophthalmic devices such as an eye tonometer, a fundus camera, and an auto reflex keratometer, and thermometers. Hereinafter, a case where the medical examination device is a slit lamp will be described as an example.

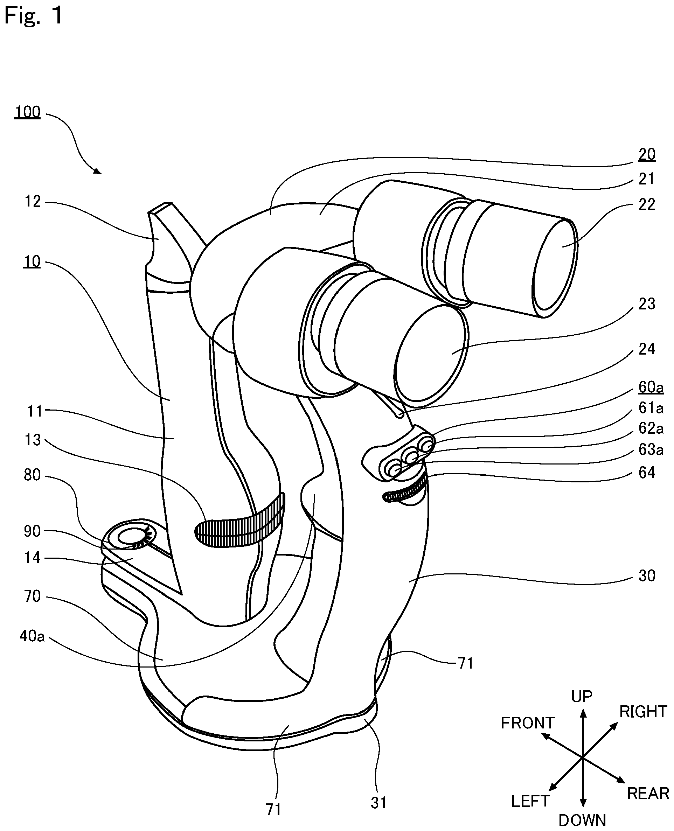

Figs. 1 to 3 are external views illustrating an example of an external appearance of a configuration of a medical examination device 100 corresponding to at least one of embodiments of the present invention. The medical examination device 100 is a device operated in a gripped state with one hand to examine an eye to be examined. As illustrated in Figs. 1 to 3, the medical examination device 100 includes an irradiation unit 10, an observation unit 20, a grip portion 30, a finger hook portion 40a, an index finger-side operation unit 50, a thumb-side operation unit 60a, a base portion 70, an irradiation angle adjustment unit 80, and a scale portion 90.

The irradiation unit 10 has a function of irradiating the eye to be examined with irradiation light. In the present example, the irradiation unit 10 has a function of irradiating the eye to be examined with slit light or spot light as irradiation light. The irradiation unit 10 includes an irradiation tube 11, an irradiation port 12, a disk operation unit 13, a swing portion 14, and a separate light source installation unit 15. Inside the irradiation tube 11, the known components for irradiating slit light as described in Patent Literature 1 cited above are arranged. In the present example, the irradiation tube 11 includes a light source, a condenser lens, a spot disk, a slit disk, and a light projecting lens inside. An example of the light source includes an LED. In the irradiation tube 11, light emitted from the light source is condensed by the condenser lens and then passes through the slit disk to generate slit light or spot light. The slit light or the spot light is incident on the irradiation port 12. The irradiation port 12 includes a light projecting prism disposed inside. The slit light or the spot light incident on the light projecting prism changes its direction to be irradiated toward the eye to be examined outside the device. The disk operation unit 13 is a dial with which an operator rotates the spot disc and the slit disc to select a length and a width of slit light to be irradiated and a diameter of spot light to be irradiated. The swing portion 14 is formed of a plate-like member of which one end portion is provided on a lower side of the irradiation tube 11 and the other end portion positioned at a predetermined distance from the irradiation tube 11 is connected to the irradiation angle adjustment unit 80, which will be described below. Note that a marker for indicating a rotation angle of the irradiation unit 10 is provided in a portion of the swing portion 14 contacting an outer circumference of the irradiation angle adjustment unit 80.

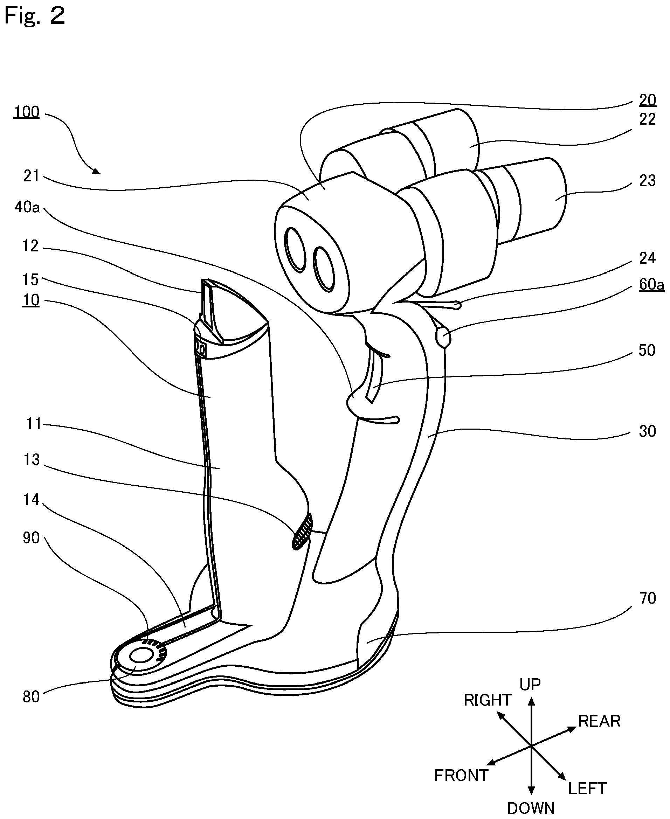

The separate light source installation unit 15 has a function of irradiating the eye to be examined with light for fluorescence observation illumination or background illumination, and is provided separately from the irradiation port 12. The separate light source installation unit 15 includes a light source installation unit for fluorescence observation illumination and a light source installation unit for background illumination. For example, a blue LED is installed as a light source in the light source installation unit for fluorescence observation illumination, and a white LED is installed as a light source in the light source installation unit for background illumination. Note that the separate light source installation unit 15 may include only one of the light source installation unit for fluorescence observation illumination and the light source installation unit for background illumination. Conventionally, light for fluorescence observation illumination or background illumination is irradiated from the same irradiation port as slit light or spot light, but it is necessary to switch between the slit or spot light and the light for fluorescence observation illumination or background illumination, and thus, it is not possible to simultaneously irradiate the slit or spot light and the light for fluorescence observation illumination or background illumination. In contrast, since the light source is directly installed in the separate light source installation unit 15 provided in the medical examination device 100, light for fluorescence observation illumination or background illumination can also be irradiated at the time of irradiating slit light or spot light. As a result, the light for fluorescence observation illumination or background illumination can be used, for example, for photographing a slit light irradiation state (e.g., photographing an anterior ocular segment of a person to be examined as a subject using background illumination) to explain the person to be examined as the subject. In addition, since the light for fluorescence observation illumination or background illumination is generated through an optical path different from the optical path of the slit light or the spot light, its irradiation range is not limited by a size of a slit turret, and the light source can be installed so that the light for fluorescence observation illumination or background illumination is illuminated in a wider range as compared with a light source in a case where light for fluorescence observation illumination or background illumination is generated through the same optical path as slit light or the spot light. In addition, as illustrated in Fig. 2, the separate light source installation unit 15 is provided so that light irradiated from the irradiation port 12 and light irradiated from the separate light source installation unit 15 are always directed in substantially the same direction. For example, the separate light source installation unit 15 rotates in the same manner as the irradiation port 12. With such a configuration, it is not necessary to separately adjust a direction of fluorescence observation illumination or background illumination at the time of adjusting an irradiation direction of slit light or spot light, resulting in an improvement in easily handling the medical examination device 100.

The observation unit 20 has a function of observing the eye to be examined with the irradiation light irradiated from the irradiation unit 10. In the present example, the observation unit 20 includes an observation casing 21, a right-eye eyepiece 22, a left-eye eyepiece 23, and a magnification lever 24. The known observation optical system described in Patent Literature 1 cited above is configured inside the observation casing 21, the right-eye eyepiece 22, and the left-eye eyepiece 23. The observation optical system is divided into an observation optical system for right eye and an observation optical system for left eye. The observation optical system for right eye is configured inside the observation casing 21 and the right-eye eyepiece 22. In addition, the observation optical system for left eye is configured inside the observation casing 21 and the left-eye eyepiece 23. For example, the observation optical system includes at least an objective lens, an eyepiece prism, a reticle lens, and an eyepiece lens. The slit light from the irradiation unit 10 is scattered in the eye to be examined and is incident on the objective lens as scattered light. The incident scattered light passes through the eyepiece prism, the reticle lens, and the eyepiece lens, and becomes light that can be observed by the operator. The magnification lever 24 is a lever that can be moved in a left-right direction to move the objective lens in a front-back direction, thereby changing a magnification at which the eye to be examined is observed.

The grip portion 30 is provided to be gripped by the operator in such a manner as to be sandwiched between a thumb and at least one of four fingers on an index finger side (hereinafter referred to as "index finger-side fingers") of one hand. The shape of the grip portion 30 is not particularly limited as long as the operator can grip the grip portion, but a substantially cylindrical shape is preferable. In the present example, as illustrated in Figs. 1 to 3, the grip portion 30 is attached to a lower side of the observation casing 21 and has a warped cylindrical shape. Hereinafter, a state in which the central axis of the grip portion 30 is oriented in a substantially vertical direction (an up-down direction in Figs. 1 to 3) will be referred to as "reference state". The direction of the central axis of the grip portion 30 illustrated in Figs. 1 to 3 is assumed to be a substantially vertical direction. Therefore, the state illustrated in Figs. 1 to 3 is the reference state.

The finger hook portion 40a is provided on the grip portion 30 to be hooked on any of the index finger-side fingers when the operator grips the grip portion 30 with one hand. Here, the index finger side refers to a side that an index finger can contact when the grip portion 30 is gripped of the side surface of the grip portion 30. The finger located on the index finger side is at least one of an index finger, a middle finger, a ring finger, and a little finger. In addition, the finger hook portion 40a being hooked on the finger means that the finger is receiving a load of the finger hook portion 40a. The shape of the finger hook portion 40a is not particularly limited as long as the finger hook portion 40a can be hooked on any of the fingers located on the index finger side. In the example illustrated in Figs. 1 to 3, the finger hook portion 40a protrudes in a flange shape from the side surface of the grip portion 30. By providing the finger hook portion 40a on the grip portion 30, the operator can grip the grip portion 30 in a state where the finger hook portion 40a is hooked on any of the fingers located on the index finger side. Therefore, the operator can be enabled to stably perform operation for examination at an ideal position of a hand gripping the grip portion 30 fixed by the finger hook portion 40a. An example of the shape of the finger hook portion will be described below.

The index finger-side operation unit 50 is used for examination operation, and is provided at a position that can be reached by a finger assumed to be used for the operation in advance as a finger of which a position is fixed by the finger hook portion 40a when the operator grips the grip portion 30 with one hand among the index finger-side fingers. The finger assumed to be used for the operation in advance is not particularly limited as long as it is any one of the index finger-side fingers, but it is preferable that the finger assumed to be used for the operation in advance is a finger other than the finger on which the finger hook portion 40a is hooked. In the present example, the index finger is assumed as a finger used for the operation. In addition, the position that can be reached by the finger refers to a position that the finger reaches by moving the finger. An example of the position that can be reached by a finger assumed to be used for the operation in advance includes a position of the grip portion 30 facing the finger assumed to be used for the operation in advance when the operator grips the grip portion 30 with one hand. The index finger-side operation unit 50 is, for example, a button, a switch, or the like. In the present example, the index finger-side operation unit 50 is a switch for causing the light source disposed in the irradiation tube 11 of the irradiation unit 10 to emit light, such that the light is irradiated from the irradiation port 12, while being while being pressed.

Further, assuming that a finger closer to the thumb than a finger receiving a load from the finger hook portion 40a is an operation finger, the index finger-side operation unit 50 may be provided at a position that can be reached by the operation finger. The finger closer to the thumb than the finger receiving the load is, for example, the index finger in a case where the finger receiving the load in the reference state is the middle finger. With such a configuration, it is possible to more stably perform operation for examination using a finger having a relatively small influence of the load from the finger hook portion 40a.

The thumb-side operation unit 60a is used for examination operation the eye to be examined, and is provided at a position that can be reached by the thumb of which a position is fixed by the finger hook portion 40a when the operator grips the grip portion 30 with one hand. The thumb-side operation unit 60a may have a plurality of operation portions. Operation members such as buttons, various switches, and dials for performing operation inputs related to the examination of the eye to be examined are provided at the operation portions. In the present example, operation buttons 61a to 63a and an operation dial 64 are provided as operation members at the operation portions of the thumb-side operation unit 60a. The operation buttons 61a to 63a are assigned, for example, various examination-related functions such as turning on/off fluorescence observation illumination or background illumination. Furthermore, the operation dial 64 is assigned, for example, a function of adjusting an amount of slit light irradiated from the irradiation port 12 of the irradiation unit 10. The operation portions of the plurality of operation buttons included in the thumb-side operation unit 60a are not particularly limited as long as the operation portions are located to be reachable by the thumb of which a position is fixed by the finger hook portion 40a when the operator grips the grip portion 30 with one hand. Examples of shapes, sizes, arrangement, and the like of the plurality of operation members included in the thumb-side operation unit 60a will be described below.

The base portion 70 is a plate-like member of which one end portion is attached onto a lower side of the grip portion 30. The irradiation angle adjustment unit 80, which will be described below, is provided at the other end portion of the base portion 70.

The irradiation angle adjustment unit 80 is a member that adjusts an irradiation angle of irradiation light by rotating the irradiation unit 10 about a rotation shaft. In the present example, the irradiation angle adjustment unit 80 is fixedly attached onto the other end portion of the base portion 70. Further, in the present example, the irradiation angle adjustment unit 80 is rotatably attached into a hole formed in the swing portion 14 at a portion spaced apart by a predetermined distance from the irradiation tube 11, with an upper surface thereof being exposed. That is, the base portion 70 and the swing portion 14 are connected to each other via the irradiation angle adjustment unit 80, and the swing portion 14 is rotatable about the irradiation angle adjustment unit 80 with respect to the base portion 70. Note that the irradiation angle adjustment unit 80 is constituted by, for example, a bearing.

The scale portion 90 provides scales each indicating a rotation angle on an upper surface of the rotation shaft. Specifically, the scale portion 90 provides scales each corresponding to an irradiation angle of irradiation light irradiated by the irradiation unit 10 in an arc shape on the upper surface of the rotation shaft. In the present example, the scale portion 90 is provided as a mark indicating a rotation angle of the swing portion 14 about the irradiation angle adjustment unit 80 in an arc shape on the upper surface of the irradiation angle adjustment unit 80 fitted to the swing portion 14. The scale portion 90 will be described in detail below.

The example of the configuration and appearance of the medical examination device 100 has been described above. Note that the medical examination device 100 may be a device in which a load balance is configured so that a center of the balance of the entire device is located on the index finger side when the grip portion is divided into two sides, the thumb side and the index finger side, by a reference plane that is a virtual plane parallel to the central axis and passes the position of the finger hook portion 40a. With such a load balance, even when the grip portion 30 is loosely gripped by a hand, the medical examination device 100 is inclined in a direction from the thumb side to the index finger side with the finger hook portion 40a as a reference. Since the inclination occurs with the finger hook portion 40a as a reference, the finger hook portion 40a remains hooked on the fingers, for example, in the reference state. As a result, even when the grip of the hand becomes loose, the state where the grip portion 30 is gripped can be maintained more easily.

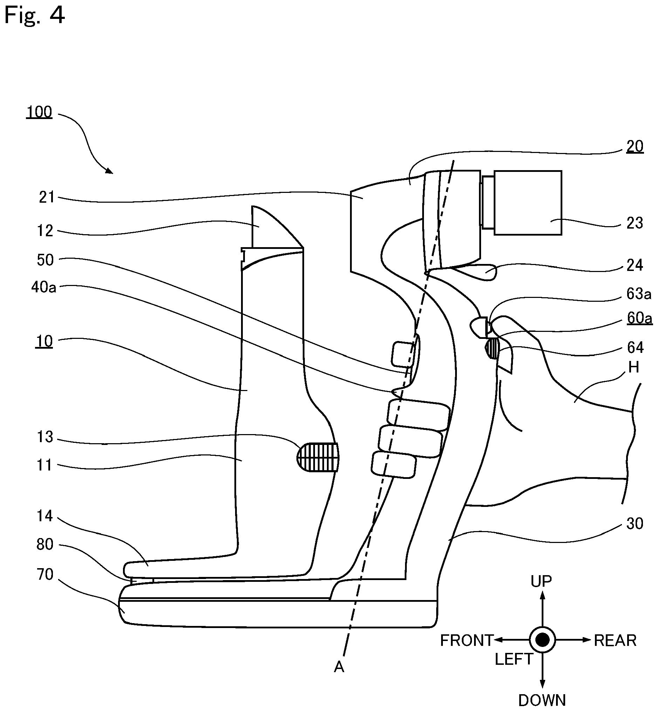

Fig. 4 is an external view when an operator grips the medical examination device 100 corresponding to at least one of embodiments of the present invention with one hand. As illustrated in Fig. 4, the grip portion 30 is gripped in such a manner as to be sandwiched between a thumb and an index finger of a hand H of the operator. The grip portion 30 is divided into an index finger side (a front side in Fig. 4) where four fingers are located and a thumb side (a rear side in Fig. 4) where one thumb is located. The finger hook portion 40a is provided on the index finger side, and is hooked on a middle finger of the hand H. That is, in Fig. 4, the middle finger of the hand H is receiving a load of the finger hook portion 40a. The index finger of the hand H is in contact with the index finger-side operation unit 50 and is ready for operation. In addition, the thumb of the hand H is in contact with the thumb-side operation unit 60a and is ready for operation. Note that a plane that passes a one-dot chain line A and divides the grip portion into two sides, the thumb side and the index finger side, is the above-described reference plane. The medical examination device 100 in the present example is a device in which a load balance is configured such that a center of the balance of the entire device is located on the index finger side when the grip portion is divided into two sides, the thumb side and the index finger side, by the reference plane.

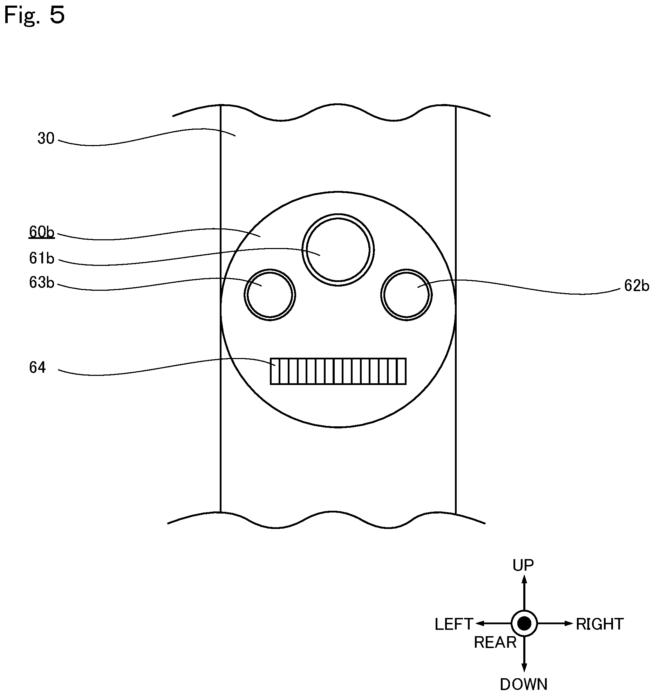

Meanwhile, all of the operation buttons 61a to 63a as the operation members provided at the operation portions of the thumb-side operation unit 60a illustrated in Fig. 1 have the same size, and are provided to be spaced apart from one another at a predetermined distance in the horizontal direction in the reference state. However, the position of the operation portions and the shape and size of the operation members are not limited thereto. Hereinafter, the operation portions and the operation members of the thumb-side operation unit will be described using operation buttons as examples with reference to Figs. 5 to 8.

Figs. 5 to 8 are explanatory diagrams for explaining operation buttons used for examination operation in the thumb-side operation unit of the medical examination device 100 corresponding to at least one of embodiments of the present invention. Note that the portion where the operation dial 64 is provided is similar to that of the thumb-side operation unit 60a in the present example, and thus description thereof will be omitted.

As illustrated in Fig. 5, a thumb-side operation unit 60b includes operation buttons 61b to 63b and an operation dial 64. Here, the operation button 61b has a different area that can be touched by an operator's finger as compared with the other operation buttons 62b and 63b. Here, the area of the operation button 61b is preferably larger than that of the operation buttons 62b and 63b by a predetermined ratio or more. With such a configuration, even when an operation button is operated by the thumb without a firm visual check, the operator can grasp a position of the thumb, or grasp which operation button the thumb is in contact with, on the basis of a difference in area between the operation buttons.

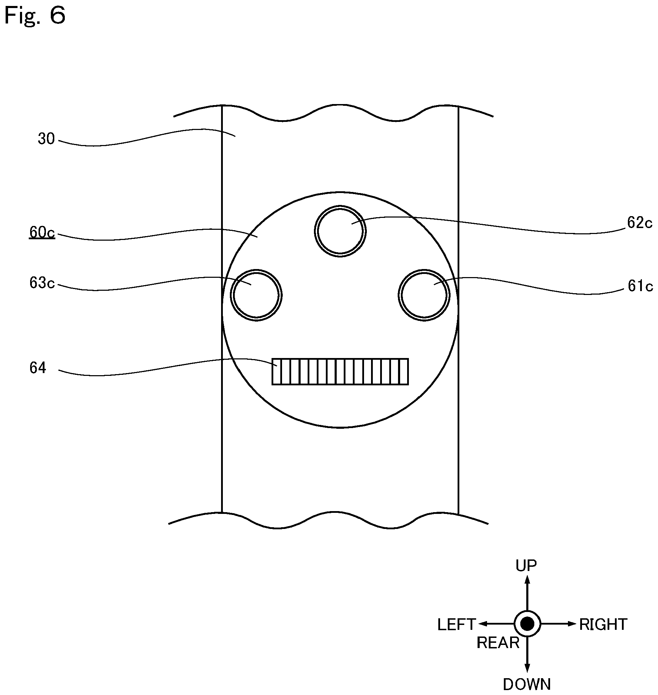

In addition, as illustrated in Fig. 6, a thumb-side operation unit 60c includes operation buttons 61c to 63c and an operation dial 64. Here, the operation buttons 61c to 63c are provided to be spaced apart from one another at a longer distance than those in the example illustrated in Fig. 1. Specifically, the operation buttons 61c to 63c are provided to be spaced apart from one another at a distance capable of preventing the operation buttons 61c to 63c from being simultaneously pressed with a size of the thumb assumed in advance. By providing the operation buttons in this manner, it is possible to prevent the operation buttons from being simultaneous operated as an erroneous operation even without a firm visual check.

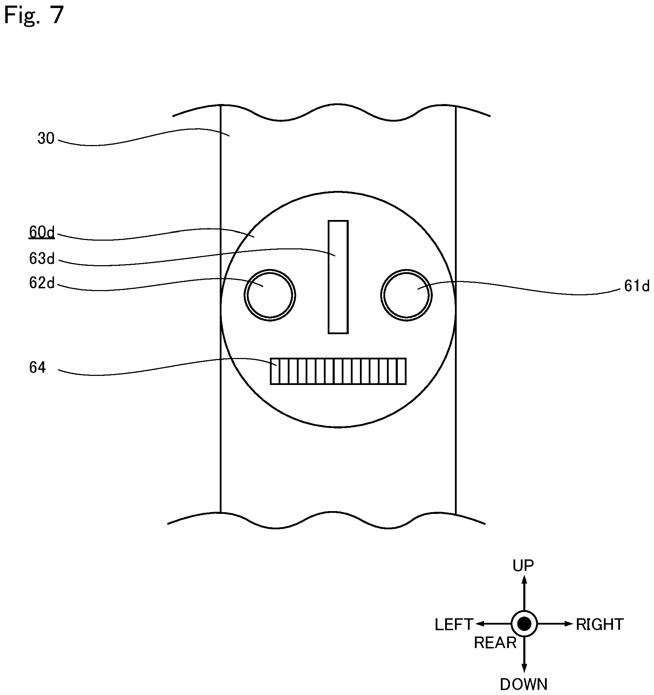

In addition, as illustrated in Fig. 7, a thumb-side operation unit 60d includes operation buttons 61d and 62d, a barrier 63d, and an operation dial 64. Here, the barrier 63d is a member having a shape to protruding outward (rearward) by a predetermined length or more, and is provided between the operation button 61d and the operation button 62d. Here, the predetermined length is appropriately determined based on a size of the operation buttons 61d and 62d and the like. Examples of the protruding shape include a convex shape and a rectangular parallelepiped shape. The barrier 63d preferably has a different shape from the other operation members provided in thumb-side operation unit 60d. By providing the barrier 63d between the operation buttons in this manner, the barrier enables the operator to ascertain a position of the thumb or avoid placing the thumb at a position where the operation buttons can be operated simultaneously, even without a firm visual check, thereby making it possible to prevent the operation buttons from being simultaneous operated as an erroneous operation.

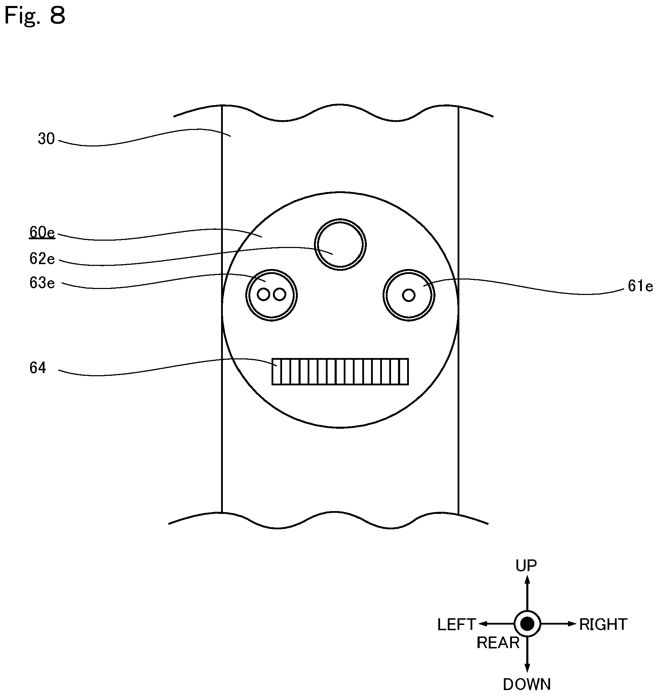

In addition, as illustrated in Fig. 8, a thumb-side operation unit 60e includes operation buttons 61e to 63e and an operation dial 64. Here, one protrusion is formed on a surface of the operation button 61e. Further, two protrusions are formed on a surface of the operation button 63e. No protrusion is formed on the operation button 62e. Note that each operation button preferably has a different number of protrusions formed thereon. By forming protrusions on the surfaces of the operation buttons as described above, the operator can grasp which operation button the thumb is in contact with, based on the number of protrusions, even without a firm visual check.

From the viewpoint of preventing inconvenience caused by simultaneously pressing the operation buttons as an erroneous operation, functions assigned to operation buttons provided at adjacent operation portions may be a combination of predetermined functions. Here, the combination of predetermined functions is preferably a combination causing a smaller influence on examination operation when the corresponding processes are executed substantially simultaneously than the other combinations. An example of the combination of predetermined functions includes a combination of a fluorescence observation illumination switching function and a photographing function. By assigning a combination of predetermined functions to operation buttons provided at adjacent operation portions in this manner, a risk can be reduced at the time of operation, and it is possible to enable the operator to execute examination operation without giving the operator stress concerning simultaneously pressing the operation buttons.

The operation portions and the operation buttons of the thumb-side operation unit have been described above using the operation buttons as examples. Of course, the shapes, the sizes, the provided operation portions, and the like described with reference to Figs. 5 to 8 can also be applied to other types of operation members such as various switches and dials.

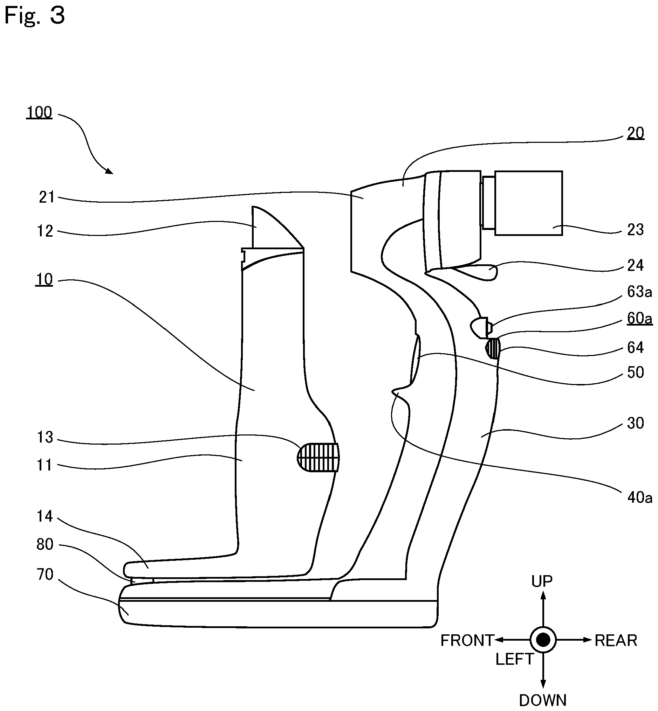

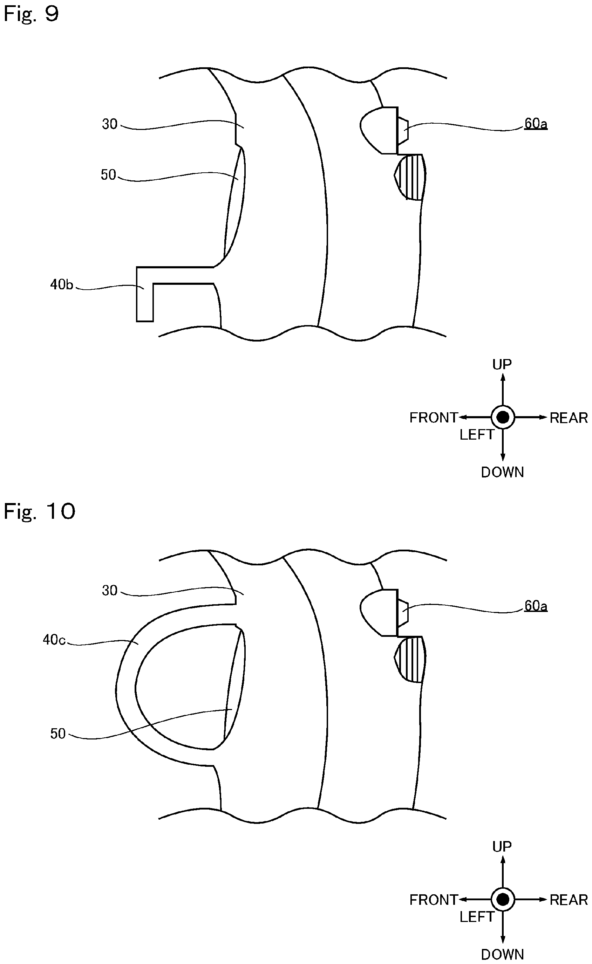

Next, examples of the shape of the finger hook portion of the medical examination device 100 will be described. As illustrated in Fig. 3, the above-described finger hook portion 40a has a shape to protrude in a direction (a forward direction) substantially perpendicular to the central axis of the grip portion 30. However, the shape of the finger hook portion is not limited thereto. Hereinafter, examples of the shape of the finger hook portion will be described with reference to Figs. 9 and 10.

Figs. 9 and 10 are explanatory diagrams for explaining examples of the shape of the finger hook portion of the medical examination device 100 corresponding to at least one of embodiments of the present invention. Hereinafter, finger hook portions having different shapes from the above-described finger hook portion 40a will be described with reference to Figs. 9 and 10.

As illustrated in Fig. 9, a finger hook portion 40b has a shape to protrude outward from the side surface of the grip portion 30 on the index finger side and bent substantially vertically in a downward direction in the reference state. Here, the shape of the finger hook portion 40b is not particularly limited as long as the finger hook portion 40b is bent substantially vertically in the downward direction in the reference state, but the finger hook portion 40b preferably has a shape to have a portion extending in a direction toward the finger receiving a load of the finger hook portion 40b, thereby restricting a movement of the finger. By taking the shape of the finger hook portion 40b, it is easy to keep the finger hook portion 40b in a hooked state on the finger, and as a result, the operator can perform examination operation more stably.

In addition, as illustrated in Fig. 10, a finger hook portion 40c has a shape to protrude outward from the side surface of the grip portion 30 on the index finger side, to be bent in a vertically upward direction in the reference state, and to be attached to the grip portion 30 on the index finger side in the vertically upward direction above the finger assumed to be used for the operation in advance in the reference state. That is, both ends of the finger hook portion 40c are attached to the index finger side of the grip portion 30. The finger hook portion 40c has a ring shape by including a part of the grip portion 30. Here, the index finger-side operation unit 50 is preferable provided between portions where both ends of the finger hook portion 40c are attached. The number of fingers assumed in advance to be put into the ring shape formed by the finger hook portion 40c may be one or more. With such a configuration, it is difficult to perform an operation input to the index finger-side operation unit 50 when a finger is not put in the ring shape formed by the finger hook portion 40c as compared with that in a case where the finger hook portion does not have a ring shape, making it possible to prevent an erroneous operation of the index finger-side operation unit 50.

The examples of the shape of the finger hook portion of the medical examination device 100 have been described above. Note that, although it has been described that one finger hook portion is provided in the medical examination device 100, a plurality of finger hook portions may be provided in one medical examination device 100. For example, in Fig. 1, it may be considered that a finger hook portion for the ring finger is added, so that the middle finger and the ring finger are hooked on the respective finger hook portions. In addition, the plurality of finger hook portions provided in the medical examination device 100 may be formed in one of the types of the above-described finger hook portions 40a to 40c and the like, or some or all of the finger hook portions may be formed in different types.

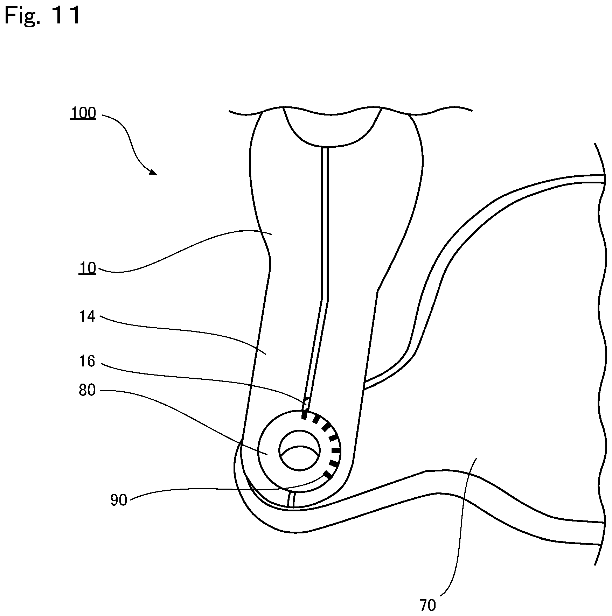

Fig. 11 is an explanatory diagram for explaining an example of the scale portion 90 of the medical examination device 100 corresponding to at least one of embodiments of the present invention. In Fig. 11, the irradiation unit 10, the base portion 70, the irradiation angle adjustment unit 80, and the scale portion 90 are shown. As illustrated in Fig. 11, the irradiation unit 10 is rotated with respect to the base portion 70 by the irradiation angle adjustment unit 80. As described above, the irradiation angle adjustment unit 80 is in a state where the upper surface thereof is exposed while being fitting into a hole formed in the swing portion 14 at a portion spaced apart by a predetermined distance from the irradiation tube 11. Since the irradiation tube 11 and the irradiation angle adjustment unit 80 are attached to the swing portion 14 at a predetermined distance as described above, when the swing portion 14 rotates about the irradiation angle adjustment unit 80, the irradiation tube 11, the irradiation port 12 provided in an upper portion of the irradiation tube 11, and the separate light source installation unit 15 rotate while changing their directions. Therefore, an irradiation angle of irradiation light irradiated from the irradiation port 12 and the separate light source installation unit 15 can be adjusted by the irradiation angle adjustment unit 80.

In addition, the scale portion 90 is provided on the upper surface of the irradiation angle adjustment unit 80. For example, a plurality of scales are provided as the scale portion 90 every predetermined rotation angle with the state of Figs. 1 to 3 as a reference. By providing the scale portion 90 on the upper surface of the rotation shaft in this manner, in order to adjust an irradiation angle, the operator can check a rotation angle by visually observing the scale portion 90 at the time of operation for examining the eye to be examined. As illustrated in Fig. 11, in order to accurately grasp a current rotation angle, the scale portion 90 may include a marker 16 for adjusting a rotation angle of the irradiation unit 10 at a portion contacting an outer circumference of the irradiation angle adjustment unit 80 in the swing portion 14. From a positional relationship between the marker 16 and a scale on the upper surface of the irradiation angle adjustment unit 80, the operator can grasp a more accurate rotation angle at the time of examination operation.

As described above, in a medical examination device having a grip portion that is grippable by an operator in such a manner as to be sandwiched between a thumb and at least one of index finger-side fingers of one hand, to examine a subject by performing operation in a state where the grip portion is gripped with the one hand, according to the present invention, a finger hook portion is provided on the grip portion to be hooked on any of the index finger-side fingers when the grip portion is gripped by the operator with the one hand. Therefore, the operator is enabled to perform stable operation for examination in a state where the hand is gripping the grip portion at an ideal position fixed by the finger hook portion.

That is, for example, even when the operator grasps the examination device without a firm visual check, the operator can grasp the device at an ideal position for operation by hooking the finger hook portion on the finger. Furthermore, even when the grip of the operator's gripping hand becomes loose, the finger hook portion remains hooked on the finger, thereby making it possible to prevent the hand from being deviated from the ideal position for the operation.

In addition, in the medical examination device according to the present invention, as illustrated in Fig. 1, the base portion 70 is provided, thereby enabling medical examination device 100 to stand by itself. The base portion 70 has a grounded surface that is grounded to a location at which the medical examination device is placed, but the grounded surface needs to have a predetermined area or more or have a shape capable of maintaining balance so that the medical examination device stably stands by itself. Here, in the present example, by devising connection between the base portion 70 and the grip portion 30 of the medical examination device 100, it is possible to achieve a shape for the operator to easily hold the medical examination device 100 without hindering the self-standing of the medical examination device 100.

Specifically, as illustrated in Fig. 1, a positional relationship between the grip portion 30 and the base portion 70 is determined such that a grip end 31, which is a lower end of the grip portion 30 of the medical examination device 100, has a shape to protrude from a base portion rear end 71 of the base portion 70 (it can also be said that the base portion rear end 71 is retracted more than the grip end 31).

With such a shape, when the operator grips the grip portion 30, a little finger side of a palm of the operator is less likely to hit the base portion 70, resulting in an effect in that the handling of the medical examination device 100 is facilitated. In particular, in a case where the grip portion 30 is held to in a state where a wrist is raised (in a state where a vector from an elbow toward the wrist is approximately upward), since the base portion rear end 71 is retracted, a space is formed for the vicinity of the base of the operator's palm to escape, making it easy to hold the grip portion 30. In addition, since the base portion rear end 71 is retracted, there is a space, making it possible to support the entire device by pushing the vicinity of the base of the operator's palm against the vicinity between the base portion rear end 71 and the grip end 31 in a state where the operator grips the grip portion 30. In addition, the medical examination device 100 illustrated in Fig. 1 has a problem that it is likely to fall backward due to the center of balance. However, in the present embodiment, since the grip end 31 protrudes, the grip end 31 bears some of the overturn prevention effect of the base portion 70, and thus, the overturn prevention effect is not impaired.

### Reference Signs List

- 100: medical examination device
- 10: irradiation unit
- 11: irradiation tube
- 12: irradiation port
- 13: disk operation unit
- 14: swing portion
- 15: separate light source installation unit
- 16: marker
- 20: observation unit
- 21: observation casing
- 22: right-eye observation unit
- 23: left-eye observation unit
- 24: magnification lever
- 30: grip portion
- 31: grip end
- 40a to 40c: finger hook portion
- 50: index finger-side operation unit
- 60a to 60e: thumb-side operation unit
- 70: base portion
- 71: base portion rear end
- 80: irradiation angle adjustment unit
- 90: scale portion

## Claims

1. A medical examination device having a grip portion that is grippable by an operator in such a manner as to be sandwiched between a thumb and at least one of four fingers including an index finger, a middle finger, a ring finger, and a little finger on an index finger side (hereinafter referred to as "index finger-side fingers") of one hand, to examine a subject by performing operation in a state where the grip portion is gripped with the one hand, the medical examination device comprising:
a finger hook portion provided on the grip portion to be hooked on any of the fingers located on the index finger side when the grip portion is gripped by the operator with the one hand.

2. The medical examination device according to claim 1, wherein
an index finger-side operation unit used for examination operation is provided at a position that is reachable by a finger assumed to be used for the operation in advance as a finger of which a position is fixed by the finger hook portion when the operator grips the grip portion with the one hand among the index finger-side fingers.

3. The medical examination device according to claim 2, wherein,
assuming that a finger closer to the thumb than a finger receiving a load from the finger hook portion is an operation finger, the index finger-side operation unit is provided at a position that is reachable by the operation finger.

4. The medical examination device according to any one of claims 1 to 3, wherein
a thumb-side operation unit used for examination operation is provided at a position that is reachable by the thumb of which a position is fixed by the finger hook portion when the operator grips the grip portion with the one hand.

5. The medical examination device according to any one of claims 1 to 4, further comprising:
an irradiation unit that irradiates the subject with irradiation light;
an irradiation angle adjustment unit that adjusts an irradiation angle of the irradiation light by rotating the irradiation unit about a rotation shaft; and
a scale portion that provides scales each indicating a rotation angle on an upper surface of the rotation shaft.

6. The medical examination device according to any one of claims 1 to 5, wherein
a load balance is configured so that a center of the balance of the entire device is located on the index finger side when the grip portion is divided into two sides, a thumb side and the index finger side, by a reference plane that is a virtual plane parallel to a central axis of the grip portion and passes a position of the finger hook portion.

7. The medical examination device according to any one of claims 1 to 6, further comprising
a base portion having a grounded surface that is grounded to a location at which the medical examination device is placed, with one end portion thereof being attached to a lower side of the grip portion, wherein
a grip end, which is a lower end of the grip portion, has a shape to protrude from a rear end of the base portion.
